# EUROPÄISCHE PATENTSCHRIFT

(11) **EP 1 073 895 B1**
(45) Veröffentlichungstag und Bekanntmachung des Hinweises auf die Patenterteilung: **28.03.2012**
(21) Anmeldenummer: 99920697.2
(22) Anmeldetag: 19.04.1999
(51) Int. Cl.: G01N 27/327, G01N 33/543, C12Q 1/00

(54) **DEXTRANBESCHICHTETE OBERFLÄCHE**
DEXTRAN-COATED SURFACE
SURFACE REVETUE DE DEXTRANE

(30) Priorität: 24.04.1998 DE 19818360
(43) Veröffentlichungstag der Anmeldung: 07.02.2001
(73) Patentinhaber: Arrayon Biotechnolgy SA, 2002 Neuchatel (CH)
(72) Erfinder: BARIE, Nicole, D-76297 Stutensee (DE); GOBET, Jean, CH-2035 Corcelles (CH); RAPP, Michael, D-69214 Eppelheim (DE); SIGRIST, Hans, CH-3309 Kernenried (CH)
(86) Internationale Anmeldenummer: PCT/EP1999/002599
(87) Internationale Veröffentlichungsnummer: WO 1999/056119

(56) Entgegenhaltungen:
- EP-A- 0 886 141
- WO-A-90/04609
- WO-A-92/21976
- US-A- 5 436 161
- BARIE, N. ET AL: "Covalent photolinker -mediated immobilization of an intermediate dextran layer to polymer-coated surfaces for biosensing applications" BIOSENS. BIOELECTRON. (1998), 13(7-8), 855-860 , XP002111079
- DATABASE CHEMABS [Online] CHEMICAL ABSTRACTS SERVICE, COLUMBUS, OHIO, US MATTHIJS, G. ET AL: "Comparative study of methodologies for obtaining.beta.-glucosidase immobilized on dextran -modified silica" retrieved from STN Database accession no. 126:56773 XP002111080 & ENZYME MICROB. TECHNOL. (1996), 19(8), 601-605 ,
- SIGRIST H ET AL: "SURFACE IMMOBILIZATION OF BIOMOLECULES BY LIGHT", OPTICAL ENGINEERING, SOC. OF PHOTO-OPTICAL INSTRUMENTATION ENGINEERS, BELLINGHAM, vol. 34, no. 8, 1 August 1995 (1995-08-01) , pages 2339-2348, XP000518229, ISSN: 0091-3286, DOI: 10.1117/12.201815

## Beschreibung

Die Erfindung betrifft eine dextranbeschichtete Oberfläche, wie sie aus O' Shannessy, D.J.; Brigham-Burke, M.; Peck, K.; Anal. Biochem. 205 (1992) 132-136 bekannt ist.

Aus S. Löfas, B. Johnsson; J. Chem. Soc. Chem. Commun. (1990) 1526-1528 ist bekannt, daß Dextrane über self-assembled Monolayers von 1,ω-Hydroxyalkylthiolen an Goldoberflächen angebunden werden können.

Einige Sensoren jedoch, wie z. B. Oberflächenwellen (SAW) Sensoren erlauben keine leitfähige Zwischenschicht, wodurch Gold als intermediäre Schicht nicht verwendet werden kann.

Solche Sensoren sind z. B. aus der DE 43 19 215 A1 bekannt.

Aufgabe der Erfindung ist es, eine Schichtung anzugeben mit deren Hilfe eine Dextranschicht auf einer Trägerschicht fixiert wird.

Gelöst wird diese Aufgabe durch die Merkmale des Patentanspruchs 1.

Die Unteransprüche beschreiben vorteilhafte Ausgestaltungen der Erfindung.

Die erfindungsgemäße Schichtung hat folgende Vorteile:
Die extrem flexible Dextran-Methode kann auf SAW-Sensoren und vielen anderen Sensoren verwendet werden.

Es können nahezu alle Proteine mit Hilfe einer standardisierte Methode (Carbodiimid-Chemie) am Dextran immobilisiert werden.

Anstelle von Dextran können auch jegliche modifizierte Dextranspezies verwendet werden, wie Carboxy-Dextran, biotyniliertes Dextran. Jede andere Art von funktionalisierten Dextran, wobei eine oder mehrere reaktive Gruppen an das Dextran gebunden sind, kann ebenfalls zum Einsatz kommen.

Anwender bisheriger Biosensorsysteme müssen sich bei der Einführung Dextran-beschichteter SAW-Biosensoren bezüglich der Sensorchemie nicht umstellen.

Außerdem ist diese Methode auf beliebige andere Systeme einfach übertragbar. Es muß nur ein einziges Protein (das BSA) modifiziert werden und nicht die Substanz selbst, die eigentlich immobilisiert werden soll.

Das zur Photoimmobilisation verwendete T-BSA dient gleichzeitig zur Blockierung unspezifischer Bindungsstellen auf der schutzbeschichteten Oberfläche.

Mit Hilfe von SAW-Sensorarrays ist es möglich, verschiedene Biokomponenten gleichzeitig zu detektieren und somit ein Screening einer ganzen Klasse von Substanzen durchzuführen, was bei den käuflichen Biosensorsystemen (z. B. BIAcore oder Iasys) aufgrund der aufwendigen optischen Methodik nicht oder nur erschwert (und kostenintensiv) möglich ist.

Die Photoimmobilisation stellt im Vergleich zur Anbindung der Dextrane über Thiole eine sehr einfache, schnelle und gut reproduzierbare Methode dar.

Die Erfindung wird im folgenden anhand eines Ausführungsbeispiels mit Hilfe der Figuren näher erläutert.

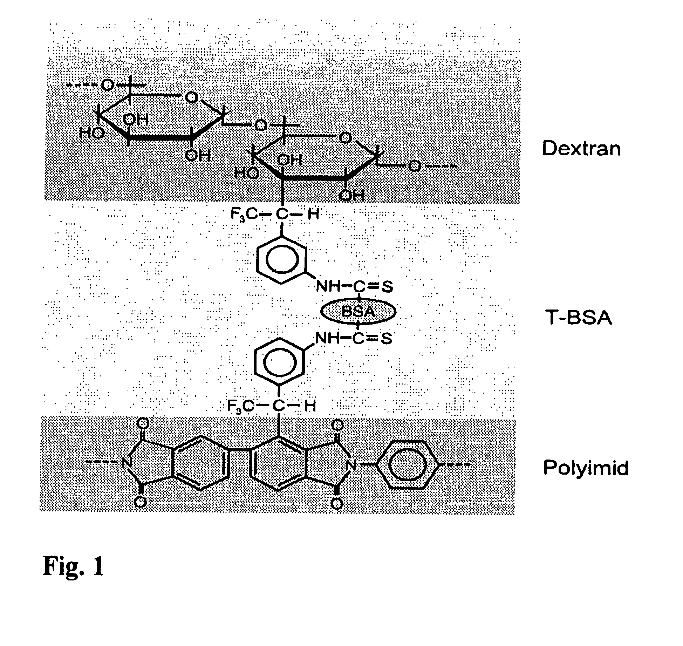

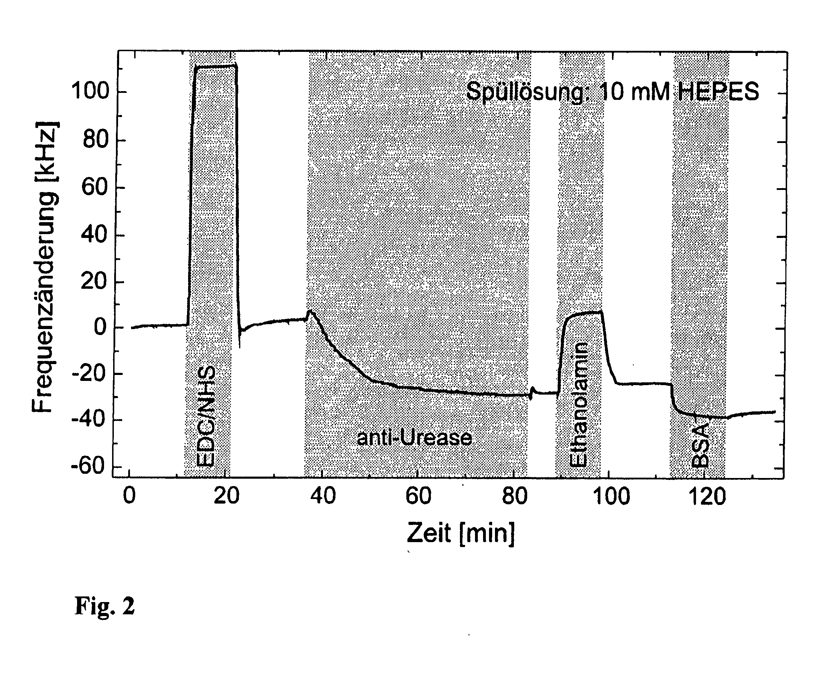

Dabei zeigt die Figur 1 den schematischen Schichtaufbau und die Figuren 2 bis 4 einige exemplarische Meßergebnisse mit beschichteten SAW- Sensoren.

### Ausführungsbeispiel:

### Schutzbeschichtungen:

Um kommerziell erhältliche SAW-Bauteile als massesensitive Transducer in der Biosensorik zum Einsatz zu bringen muß die SAW-Oberfläche mit einer biosensitiven Schicht aus Proteinen belegt werden, die dann die entsprechenden Analyt-Moleküle in der Probe nachweisen.

Diese Reaktionen erfolgen in wäßrigen Medien.

Der Einsatz in diesen wäßrigen Medien und auch die erforderlichen Immobilisationsprozeduren erfordern jedoch einen Schutz der auf dem Bauteil vorhandenen Strukturen und Bonddrähte aus Aluminium, da diese ansonsten den jeweiligen chemischen Bedingungen nicht standhalten.

Aus der DE 196 18 82 ist eine dünne Beschichtung des kompletten Bauteils mit Polyimid durch Spin-Coating bekannt, das auch schon eine relativ gute Schutzwirkung aufweist.

Nach Betreiben der Bauteile in sauren bzw. alkalischen Medien kann es jedoch zu erhebliche Korrosionen der Strukturen kommen.

Eine verbesserte Schutzwirkung kann durch eine Beschichtung mit einem dünnen Film von Parylen erhalten werden.

Parylen ist eine Gruppenbezeichnung für thermoplastische Polymere mit über Ethylen-Brücken in 1,4-Position verknüpften Phenylen-Resten. Parylene sind unterhalb ihrer Schmelzpunkte lösungsmittelbeständig, besitzen hervorragende dielektrische Eigenschaften und sind ausgezeichnete Barrierekunststoffe. Sie werden hauptsächlich verwendet als Zwischenschichten für Isolatoren, zur Passivierung von Halbleitern und kraterfreien Beschichtung von gedruckten Leiterplatten.

Hergestellt werden sie durch dehydrierende Dimerisierung von p-Xylol zum Paracyclophan über intermediär sich bildendes 1,4-Chinodimethan, das bei Kondensation aus der Gasphase auf geeigneten Substraten zu dünnen Filmen aus Poly(p-xylylen) polymerisiert.

Neben Poly(p-xylylen) sind u. a. noch die aus den entsprechenden p-Xylol-Derivaten zugänglichen Parylene Poly(2-chlor-p-xylylen)e und Poly(dichlor-p-xylylen)e für eine Beschichtung geeignet.

Die parylenbeschichteten Bauteile weisen gegenüber polyimidbeschichteten Bauteilen eine Reihe von Vorteilen auf.

Die zusätzliche Bedämpfung durch die aufgebrachte Polymerschicht ist gering, der Betrag bei Parylen und Polyimid ungefähr gleich. Die schutzbeschichteten Bauelemente weisen Dämpfungen von -2.5 bis -3.0 dB (bei einem Phasendurchgang von -10°) auf.

Die Reproduzierbarkeit der Parylenbeschichtung ist deutlich besser als die Beschichtung der SAW-Bauteile mit Polyimid.

Der Parylenfilm ist im Gegensatz zum Polyimidfilm extrem glatt, was bei der Verwendung von SAW-Bauteilen sehr wichtig ist, da die Ausbreitung der Oberflächenwelle durch Unregelmäßigkeiten der Oberfläche gestört wird und es damit zu zusätzlichen Dämpfungsverlusten und zu verringerter Empfindlichkeit kommt.

Die Schutzbeschichtung soll das SAW-Bauteil vor dem korrosiven Angriff durch wäßrige Lösungen, Säuren, Basen und aggressive Reagenzien schützen.

Bei polyimidbeschichteten Bauteilen war schon nach wenigen Minuten eine Gasentwicklung vor allem an den Kontaktstellen der Bonddrähte mit den Bondpads zu beobachten, die auf einen korrosiven Angriff der Säure auf das Aluminium schließen ließ. Nach ca. 15 - 30 min kam es dann zur vollständigen Zerstörung der Bonddrähte und zu einem deutlich erkennbaren Angriff auf die Interdigitalstruktur.

Dagegen konnte bei parylenbeschichteten Bauelementen auch nach mehrstündiger Einwirkzeit und sogar nach Erhitzen des Sensors in der Säure kein Angriff beobachtet werden. Der Parylenfilm weist damit einen ausgezeichneten Schutz des Bauteils vor korrosiven Angriffen auf.

### Photoimmobilisation des Dextrans:

Ein schematischer Schichtaufbau bei der Immobilisation von Biomolekülen an dextranbeschichteten SAW-Sensoroberflächen durch Coimmobilisation mit photoaktivem T-BSA wird in Fig. 1 gezeigt. Dabei ist Dextran kovalent an der schutzbeschichteten Sensoroberfläche durch Photo-Coimmobilisation mit Hilfe von T-BSA angebunden.

Zu dieser kovalenten Anbindung werden folgende Schritte durchgeführt:
Mischung aus T-BSA und Dextran auf den schutzbeschichteten Sensor aufbringen,
Inkubation 60 min bei Raumtemperatur,
   2 h im Vakuum trocknen (10⁻³ mbar)
   45 min Belichtung mit einer Hg-Dampflampe.

T-BSA wird aus Rinderserumalbumin (BSA) hergestellt, indem es mit einem Diazirin-Derivat, dem 3-Trifluoromethyl-3-(m-isothiocyano-phenyl)-diazirin (TRIMID), umgesetzt wird und somit an den Aminogruppen der Lysinseitenketten das photoaktive TRIMID eingeführt wird (M. Dolder, H. Michel, H. Sigrist; J. Prot. Chem. 9 (1990) 407-415 und Sigrist, H.; Mühlemann, M.; Dolder, M.; J. Photochem. and Photobiol., B 7 (1990) 277-287). Pro Molekül BSA können auf diese Weise bis zu 7 TRIMID-Gruppen eingeführt werden. Bestrahlt man nun das T-BSA mit Licht der Wellenlänge 348 nm, so spaltet der Diazirin-Ring Stickstoff ab und es entsteht ein Triplett-Carben. Dieses Triplett-Carben ist sehr reaktiv und insertiert in eine Vielzahl chemischer Bindungen. Bei der Coimmobilisation dient das T-BSA als Photovernetzer, d. h. das T-BSA wird zusammen mit dem Dextran auf das schutzbeschichtete SAW-Bauteil aufgebracht. Da - wie oben erwähnt - pro BSA-Molekül bis zu 7 TRIMID-Einheiten vorhanden sind, können die bei der Bestrahlung gebildeten Triplett-Carbene dann sowohl eine Verknüpfung mit der Schutzschicht als auch mit dem Dextran bewirken. Damit wird das Dextran kovalent an die Oberfläche angebunden.

Die Coimmobilisation von Dextran über T-BSA weist verschiedene Vorteile auf.

Zum einen handelt es sich hierbei um eine sehr einfache und schnelle Prozedur. Das T-BSA wird mit dem gelösten Dextran gemischt und auf das schutzbeschichtete Bauteil aufgebracht. Dann wird 2 h lang das Wasser im Vakuum abgezogen, damit die später bei der Belichtung gebildeten Carbene auch tatsächlich mit Polymer und Dextran reagieren und nicht mit dem umgebenden Wasser abreagieren. Nachdem das Wasser möglichst quantitativ entfernt ist, wird 45 min mit einer Hg-Dampflampe belichtet. Damit hat man dann also nach maximal 4 Stunden das Dextran kovalent an die Oberfläche angebunden.

### Immobilisation von Proteinen am Dextran und Beobachtung der Immunoreaktion:

Um einen Biosensor zu erhalten, muß das gewünschte rezeptive Biomolekül am Dextran immobilisiert werden. Eine einfache und schnelle Anbindung nahezu beliebiger Biomoleküle ist mit Hilfe der folgenden Prozedur möglich (Johnsson, B.; Löfas, S.; Lindquist, G.; Anal. Biochem. 198 (1991) 268-277).
1) Das durch Photoimmobilisation an der schutzbeschichteten Oberfläche angebundene carboxymethylierte Dextran wird in einer 1-Schritt-Aktivierung durch N-(3-Dimethylaminopropyl)-N'-ethylcarbodiimid (EDC) und N-Hydroxysuccinimid (NHS) in NHS-Ester umgewandelt. Die NHS-Ester sind sehr aktiv und haben eine Halbwertszeit auf der Sensoroberfläche von etwa 15 Minuten. Wird nun ein Biomolekül zugegeben, so bindet dieses über eine seiner Aminogruppe unter Abspaltung von N-Hydroxysuccinimid am Dextran an. Bei der 1-Schritt-Aktivierung werden etwa 30 bis 40 % der Carboxygruppen des Dextrans in NHS-Ester überführt, daher ist nach der Anbindung der Biomoleküle im allgemeinen noch immer eine Vielzahl von aktiven NHS-Estergruppen am Dextran vorhanden. Da aber beispielsweise im Fall eines immobilisierten Antikörpers bei der Immunoreaktion das Antigen durch den Antikörper gebunden und nicht über den NHS-Ester kovalent angebunden werden soll, müssen die überschüssigen NHS-Ester desaktiviert werden. Dies geschieht mit 1 M Ethanolamin. Dabei werden die NHS-Ester in 2-Hydroxy-Ethanamid-Derivate überführt und man erhält so schließlich den fertigen Biosensor.
2) Die Fig. 2 zeigt beispielhaft eine Immobilisation mit Sensorantwort.
   Der Sensor wird zunächst mit 10 mM HEPES-Puffer (pH 7.5) gespült, um die Basislinie aufzunehmen. Die 1-Schritt-Aktivierung erfolgt mit einer frisch zubereiteten Mischung aus 100 mM NHS und 400 mM EDC. Diese Lösung hat eine Leitfähigkeit von 14.4 mS/cm, was gegenüber den 550 µS/cm des HEPES-Puffers eine deutliche Leitfähigkeitserhöhung darstellt. Der Sensor reagiert darauf mit einer Frequenzzunahme von etwa 110 kHz. Anschließend wird wieder mit HEPES-Puffer gespült und dann mit der Lösung des zu immobilisierenden Biomoleküls beprobt. Das Biomolekül - in diesem Fall monoklonale Antikörper gegen Urease - liegt in einem 10 mM Acetatpuffer bei pH 5.0 vor. Man erkennt eine Frequenzabnahme, was auf eine Massenzunahme auf der Sensoroberfläche durch die Immobilisation des Biomoleküls zurüchzuführen ist. Man erkennt am Sensorverhalten die langsame Einstellung des Gleichgewichts, die nach etwa 45 bis 50 Minuten erreicht ist. Daraufhin wird wiederum mit HEPES-Puffer gespült und anschließend die überschüssigen NHS-Estergruppen durch Ethanolamin desaktiviert. Als letzter Schritt zur Präparation des Biosensors wird mit 4 mg/ml BSA beprobt, um die unspezifischen Bindungsstellen zu blocken.

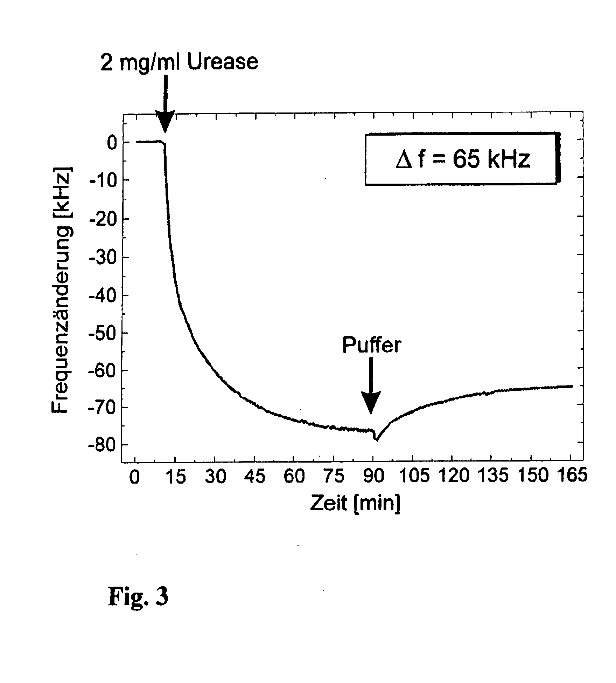

3) Die Fig. 3 zeigt die Beobachtung einer Immunoreaktion.
   An einem so vorbereiteten Sensor wurden nun erste Versuche zur Beobachtung der Immunoreaktion durchgeführt. Dazu wurde ein mit monoklonalen Antikörpern gegen Urease beschichteter Sensor zunächst mit 10 mM HEPES-Puffer (pH 7.5) gespült und die Basislinie aufgenommen. Anschließend wurde mit einer Lösung von 2 mg/ml Urease beprobt. Man erkennt eine Frequenzabnahme, die nach etwa 90 Minuten beendet ist. Nach dem Rückspülen mit HEPES-Puffer wurde eine Frequenzänderung von etwa 65 kHz erhalten, die auf die Immunoreaktion zwischen den immobilisierten Antikörpern und dem Antigen Urease zurückzuführen ist.

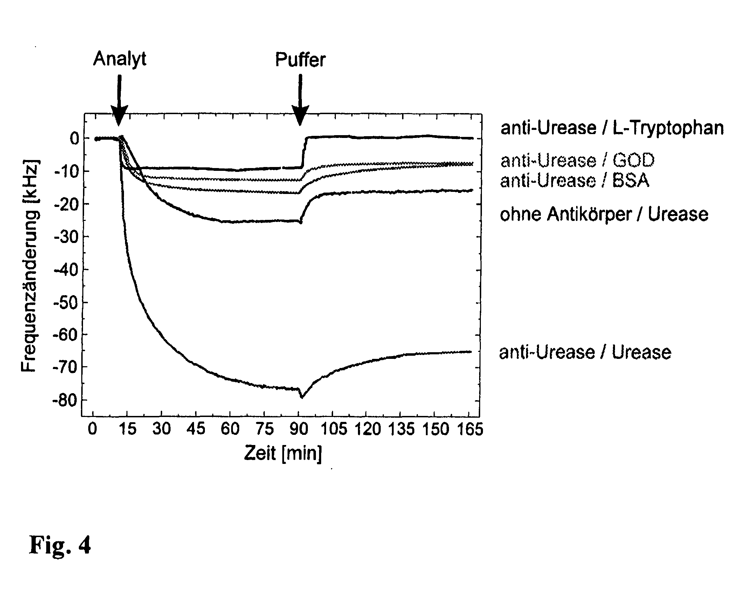

Die Fig. 4 zeigt einige Kontrollversuche mit nicht-spezifischen Antigenen.

Es wurden im weiteren mehrere Kontrollexperimente durchgeführt. Dazu wurden gleichbehandelte Sensoren mit nicht-spezifischen Antigenen beprobt und die Sensorantwort beobachtet. Bei der Beprobung eines solchen Sensors mit immobilisierten monoklonalen Antikörpern gegen Urease mit L-Tryptophan ist keine Frequenzänderung zu beobachten. Die schnelle aber reversible Frequenzänderung bei der Beprobung mit Analyt bzw. beim Zurückspülen mit Puffer ist auf eine unterschiedliche Leitfähigkeit beider Lösungen zurückzuführen. Bei der Beprobung mit Glucoseoxidase (GOD) bzw. mit BSA erhält man jeweils eine Frequenzänderung von etwa 8 kHz. Ein Sensor, der keine immobilisierten Antikörper an der Dextranmatrix enthält, machte bei Beprobung mit Urease eine Frequenzänderung von etwa 15 kHz, die auf die unspezifische Reaktion von Urease zurückzuführen ist. All diese Frequenzänderungen sind aber deutlich niedriger als die der spezifischen Immunoreaktion zwischen Urease und Antikörpern gegen Urease, die zu 65 kHz bestimmt wurde.

## Patentansprüche

1. Dextranbeschichtete Oberfläche auf einem Träger, **dadurch gekennzeichnet, dass** die Verbindung von Dextran und der Trägeroberfläche durch einen Photolinker vermittelt wird, wobei die Dextranbeschichtung durch Coimmobilisation eines Gemisches aus Dextran und dem Photolinker T-BSA erfolgt.

2. Dextranbeschichtete Oberfläche nach Anspruch 1, **dadurch gekennzeichnet, dass** die Photo-Coimmobilisation mit Hilfe von 3-Trifluoromethyl-3-(m-isothiocyanophenyl)-diazirin (TRIMID) modifiziertem T-BSA erfolgt.

3. Dextranbeschichtete Oberfläche nach Anspruch 1 oder 2, **dadurch gekennzeichnet, dass** die Trägeroberfläche mit einem Polymerfilm beschichtet ist.

4. Dextranbeschichtete Oberfläche nach einem der Ansprüche 1 bis 5, **dadurch gekennzeichnet, dass** der Polymerfilm aus Polyimid oder Poly-(p-xylylen) ist.

5. Dextranbeschichtete Oberfläche nach einem der Ansprüche 1 bis 4, **dadurch gekennzeichnet, dass** die Trägeroberfläche die Oberfläche eines massesensitiven Sensors ist.

6. Dextranbeschichtete Oberfläche nach einem der Ansprüche 1 bis 5, **dadurch gekennzeichnet, dass** der massesensitive Sensor ein Bauelement ist, das akustische Oberflächenwellen (SAW) nutzt.

7. Dextranbeschichtete Oberfläche nach einem der Ansprüche 1 bis 4, **dadurch gekennzeichnet, dass** die Trägeroberfläche die Oberfläche eines optischen oder elektrochemischen Sensors ist.

## Claims

1. A dextran-coated surface on a carrier, wherein the connection of dextran and the carrier surface is formed by a photolinker, the dextran coating being effected by co-immobilisation of a mixture of dextran and the photolinker T-BSA.

2. A dextran-coated surface according to Claim 1, wherein the photo-co-immobilisation is effected with the aid of 3-trifluoromethyl-3-(m-isothiocyanophenyl)-diazirine (TRIMID) modified T-BSA.

3. A dextran-coated surface acording to claim 1 or 2, wherein the carrier surface is coated with a polymer film.

4. A dextran-coated surface according to any one of claims 1 to 5, wherein the polymer film consists of polyimide or of poly-(p-xylylene).

5. A dextran-coated surface according to any one of claims 1 to 4, wherein the carrier surface is a surface of a mass-sensitive sensor.

6. A dextran-coated surface according to any one of claims 1 to 5, wherein the mass-sensitive sensor is a component using surface acoustic waves (SAW).

7. A dextran-coated surface according to any one of claims 1 to 4, wherein the carrier surface is a surface of an optical or electrochemical sensor.

## Revendications

1. Surface revêtue de dextrane sur un support, **caractérisée en ce que** la liaison du dextrane et de la surface du support est obtenue par l'intermédiaire d'un agent de photo-réticulation, le revêtement de dextrane s'effectuant par co-immobilisation d'un mélange de dextrane et de l'agent de photo-réticulation T-BSA.

2. Surface revêtue de dextrane conforme à la revendication 1, **caractérisée en ce que** la photo co-immobilisation est effectuée à l'aide de T-BSA modifiée par la 3-trifluorométhyl-3 (m-isothiocyanophényl) - diazirine (TRIMID).

3. Surface revêtue de dextrane conforme à la revendication 1 ou 2, **caractérisée en ce que** la surface du support est revêtue d'un film polymère.

4. Surface revêtue de dextrane conforme à l'une des revendications 1 à 5, **caractérisée en ce que** le film polymère est un film de polyimide ou de poly-(p-xylylène).

5. Surface revêtue de dextrane conforme à l'une des revendications 1 à 4, **caractérisée en ce que** la surface du support est la surface d'un capteur sensible à la masse.

6. Surface revêtue de dextrane conforme à l'une des revendications 1 à 5, **caractérisée en ce que** le capteur sensible à la masse est un composant qui utilise des ondes acoustiques de surface (SAW).

7. Surface revêtue de dextrane conforme à l'une des revendications 1 à 4, **caractérisée en ce que** la surface du support est la surface d'un capteur optique ou électrochimique.
